# EUROPEAN PATENT APPLICATION

(11) **EP 4 464 354 A1**
(43) Date of publication of application: **20.11.2024**
(21) Application number: 23173333.8
(22) Date of filing: 15.05.2023
(51) Int. Cl.: A61M 11/00, A61B 5/00, A61M 15/00, A61M 16/00, G16H 20/00

(54) **METHOD OF OPERATING AN INHALATION DEVICE, INHALATION DEVICE AND INHALATION DEVICE SYSTEM FOR PROVIDING INHALATION PROCESS/BREATH GUIDING**

(71) Applicant: Pari Medical Holding GmbH, 82319 Starnberg (DE)
(72) Inventor: FIEBIG, David, 81479 München (DE); FUCHS, Carola, 82061 Neuried (DE); FINKE, Matthias, 82152 Planegg (DE); HEINE, Benjamin, 80687 München (DE); HOFFMANN, Tobias, 86938 Schondorf am Ammersee (DE); BUCHNER, Simon, 85241 Hebersthausen (DE); STÖCKL, Carolin, 80331 München (DE); REINHART, Markus, 86919 Utting (DE)
(74) Representative: Hoffmann Eitle

(57) **Abstract**

The present disclosure and invention relates to a method of operating an inhalation device, an inhalation device and an inhalation device system for providing inhalation process/breath guiding. The devices and procedures provide information about an ongoing inhalation process to the user/ patient and further provide inhalation process/breath guiding information so as to enable the use of less patient to improve an ongoing inhalation process if it is found to be non-optimal.

## Description

### TECHNICAL FIELD

The present disclosure and invention relates to a method of operating an inhalation device, and inhalation device and an inhalation device system all preferably for providing inhalation process/breath guiding for a user, patient, caregiver, and/or physician.

Further comprised in the present disclosure and invention is an inhalation method that is optionally related to the above-mentioned method of operating an inhalation device, the inhalation device, and an inhalation device system. Also comprised is an optionally related computer program. Also all preferably for providing inhalation process/breath guiding for a user, patient, caregiver, and/or physician.

### BACKGROUND

With existing medical inhalation devices and nebulisers, a patient breathes as he or she thinks is good and normally gets no feedback and information about the ongoing inhalation process. So, currently there may be a lack of monitoring of an ongoing inhalation process and at the same time an opportunity to provide inhalation or breath guidance is missed. If an inhalation process would be monitored while it is conducted and information relating to a pertinent inhalation process parameter output, then a user/patient could be put in a position to improve the inhalation process while it is conducted.

EP 2 797 652 A1 and US 10 471 122 disclose inhalation devices configured to output signals that to some extent inform a user/patient about aspects of an ongoing inhalation process.

### SUMMARY

### Objective

It is an objective of the present disclosure and invention to provide devices and procedures that allow a user/patient and a physician or caregiver to be given information about an ongoing inhalation process that can in turn provide a basis for the user/patient to improve the inhalation process while the process is conducted. Hence, it is desired to provide devices and procedures that provide inhalation process or breath guiding with respect to an ongoing inhalation process using a medical inhalation device.

### Solution

The above objective is achieved and the above-mentioned advantages realized by the devices and procedures outlined in the present disclosure and invention, in particular the method of operating an inhalation device, the inhalation device and inhalation device system described herein below. Preferably, the present invention is further defined by the appended independent claims, whereas the dependent claims define optional features and distinct embodiments.

### Advantages

Based on the present disclosure and invention, a user/patient of an inhalation device and a physician or caregiver are put in a position to assess whether an ongoing inhalation process is performed well. If deficits or room for improvement is identified during the ongoing inhalation process, then this will be accordingly communicated to the user/patient or physician or caregiver and gives the opportunity for "live" improvements during the ongoing inhalation process.

The user/patient then acts more confidently during the ongoing inhalation process and potentially also feels somewhat safer in performing it. In addition, the present therapy quality can be significantly improved since the user/patient or physician/caregiver is informed about deficits and additionally provided with indications how to improve an ongoing inhalation process. In clinical trials, it can thus be contributed to an improved inhalation process that is closer to prescribed parameters and that thus the clinical trials are meaningful.

### DETAILED DESCRIPTION

The present disclosure and invention comprise a method of operating an inhalation device that includes: a sensing unit configured to monitor an inhalation process, to measure inhalation process parameters and to collect data of and during the inhalation process, and a control unit configured to receive and process the data collected by the sensing unit and to generate a control signal for a signalling device, wherein the method comprises:
monitoring an inhalation process comprising measuring inhalation process parameters and collecting data of and during the inhalation process, generating and sending a control signal based on the collected data to a signalling unit configured to output a signal, preferably an optical, acoustic and/or a tactile signal, wherein in response to said control signal, the signalling unit outputs: a first signal if and whenever a first pre-set threshold value of an inhalation process parameter is underrun or exceeded during the inhalation process and a second signal if and whenever a second pre-set threshold value of an inhalation process parameter, that is different from the first pre-set threshold value, is underrun or exceeded during the inhalation process and/or a third signal that is indicative of one or more inhalation process parameters and continuously output during part or all of the inhalation process.

The expression "inhalation device" as used in the present disclosure and invention relates to a medical device, i.e., a medical inhalation device that is used for the prevention and treatment of respiratory malfunctions and diseases in humans or animals, but also for improving wellbeing and as prophylaxis. Said device, in line with the skilled person's common understanding, at least comprises a mouthpiece-part through which a user/patient is able to or inhales a suitably prepared medicament according to a prescription of a physician or any other suitable agent including salt water and non-prescription medicaments and any type of aerosols. "Nebulizer" devices are also embraced. The expression "inhalation device" in fact embraces all devices suitable and configured for such functionality and purpose, preferably in the medical field or for wellbeing and prophylaxis.

An "inhalation process" is a process carried out using the above explained "inhalation device" and is conventionally a process in which a user/patient inhales a medicament or any suitable agent by means and through the inhalation device for a certain amount of time and during several breath cycles. The terminology "inhalation process" is a generic term that embraces several complete breaths or breath cycles, respectively, i.e., inhalations and exhalations per time period. In the context of the present disclosure and invention, the inhalation part thereof takes centre stage since it is the part of the breath/breath cycle with which a medicament or agent is inhaled and administered and thus preferably most important to be monitored and optimized. Monitoring and optimization of exhalation is by no means included here and fully embraced by the present disclosure and invention where needed. Said inhalation process usually has a beginning at which the inhalation starts and has an end at which inhalation stops. Therebetween a certain amount of time lapses in which the user/patient performs a certain number of breath cycles, i.e., inhalation and exhalation cycles depending on the specifics of the respective therapy or treatment, preferably at least 3 to 5 breaths/breath cycles. The totality of the time passed and/or the breath cycles defines the inhalation process as such and in its totality and as indicated above include inhalation and exhalation as part of a natural breath/breath cycle. The present disclosure and invention preferably comprises that the inhalation process to be monitored is only part of the totality of the time elapsed and the breath cycles taken. The inhalation process is preferably monitored throughout by the present procedures and devices. Preferably, although in principle possible, the concepts of the present disclosure and invention are not applied to "MDI"-processes ("metered-dosed inhalation"), which do not represent an inhalation process, but normally only a single inhalation and agent burst.

The "sensing unit" is an entity that is configured to monitor an inhalation process and collect and optionally store data with respect thereto. Said "sensing unit" embraces all suitable means for that and, for example, sensors that are suitably configured to measure pertinent inhalation parameters. The sensing unit is optionally provided and implemented within the inhalation device itself.

The "control unit" is an entity to be understood broadly and is an "IPO" (input-process-output) means that it is configured to receive data, for example, from the sensing unit, process said data, optionally store the processed data and output said data in order to instruct and control other entities either of the inhalation device itself or externally connected ones. A practical example for such control unit is a microprocessor or microcomputer with respectively configured software stored and executed thereon. The control unit is optionally provided and implemented within the inhalation device itself. Alternatively, the control unit may also be part of an external device similar to the respective option for the signalling unit. The explanations given with respect thereto below then also apply to the control unit.

Equally broadly is the definition of the "signalling unit", which is an entity that is configured to output a signal/information that is preferably recognizable by human senses, for example, audible, visible and tactile signals. As will be detailed below, said signalling unit may be integrated into the inhalation device itself and/or is part of an external entity. A more elaborate example of the signalling unit is an electronic display device, but also embraces simpler lights, speakers and/or vibrators. The first signal may be a single signal of a single type but could also be a signal sequence combined of various different signal types. Likewise, the second or third or fourth signal may be a single signal or can also be a signal sequence. Preferably, the first signal, the second signal, the third signal and the fourth signal are distinguishable and different from each other, such that the user/patient can readily identify the different meanings of the signals and different scenarios encountered during an ongoing inhalation process. The first signal, the second signal and the third signal are representative of or part of an inhalation process/breath guidance or feedback function provided by the present procedure and devices. Said inhalation process/breath guidance may be limited to only one of the first signal, the second signal and the third signal, but can also include more detailed information, for example, in graphical or written or voice form to be output by the signalling unit.

The operation method according to the present disclosure and invention, in particular the one outlined above, epitomizes a number of relevant and desired functionalities. One of said functionalities is the output of a bespoke signal, for example, the first signal in a case whenever the monitoring of an ongoing inhalation process indicates that one or more pertinent inhalation parameters have underrun or exceeded a related first pre-set threshold value for said one or more pertinent inhalation parameters. "Pre-set" here means that the threshold value is set and embodied in the procedures and devices before an inhalation process to be monitored is started. Optionally, the threshold values may experience dynamic adaption during the inhalation process. The parameter monitoring is done consistently throughout an ongoing inhalation process and thus implies that the bespoke signal may be output several times throughout the ongoing inhalation process. The first threshold value is a parametric value that is representative of the borderline between the inhalation process parameters being optimal or non-optimal. Depending on the individual inhalation process parameters underrunning or exceeding the threshold value means that the inhalation parameters fall into a non-optimal range and that inhalation is thus not performed optimally with respect thereto. The user/patient is accordingly informed by the output of the first signal and could then counteract. The first signal can basically be seen as a first information or even an alarm for the user/patient or the accompanying physician or caregiver that the inhalation process is non-optimal. The second signal is output in cases where a second pre-set threshold value of one or more inhalation process parameters is underrun or exceeded, wherein said second pre-set threshold value is different to the first one and indicative of a worsening of the inhalation process. Preferably, the second pre-set threshold value is numerically located "deeper" within a non-optimal inhalation process parameter realm. Accordingly, the second signal is output to the user/patient indicating that the quality of the ongoing inhalation process has worsened. Like the first signal, the second signal can also be considered to be an information or even alarm for the user/patient or the accompanying physician or caregiver that the inhalation process is non-optimal, but with an increased degree of urgency. In essence, the first signal and the second signal form a signal, information and/or preferably alarm cascade, wherein the first signal is issued at the onset of an ongoing inhalation process developing non-optimally, the second signal is a more assertive signal, information or preferably alarm that indicates that the quality of the inhalation process has worsened as compared to a stage in the inhalation process where the first signal was output. Described differently, if a user/patient of a medical inhalation device started performing the inhalation process in a non-optimal way, then first the first signal is issued as an initial information or alarm. If and whenever the user/patient does not improve the inhalation process and the respective inhalation process parameters worsen then the second signal is output indicating to a user/patient that there is a certain need and urgency for improving the inhalation process with respect to one or more pertinent inhalation process parameters.

In addition to the above scenario, where the first signal and the second signal represent a worsening of the inhalation process, the present disclosure and invention optionally also embraces that the first signal and the second signal are simple indicators of the degree that a pre-set threshold value is underrun or exceeded. A low degree of deviation, i.e., a deviation from the first pre-set threshold value will be notified by the first signal. A higher degree of deviation, i.e., a deviation from the second pre-set threshold value will be notified by the second signal. Hence, the signal output depends on the degree of deviation from an inhalation process parameter threshold that separates optimal and non-optimal inhalation.

The skilled reader will naturally understand that the signals and signal outputs are preferably finite and not continuous, which means that they will inform/alert the user/patient and then switch off but can also be continuous. The different options may depend on the signalling device. However, the underlying pertinent inhalation process parameters will be continuously monitored during the ongoing inhalation process and if the pre-set threshold values will be underrun or exceeded again, then the respective signals will be output again or, if continuous, maintained. Preferably, only the first signal is output if and whenever the user/patient does not underrun or exceed the second pre-set threshold value. Practically, this means if the user/patient is starting to perform the inhalation process non-optimally, but then stays stable in said non-optimal stage or even improves it, the second signal does not need to be output since no further worsening to the degree that the second pre-set threshold value is underrun or exceeded occurs. So basically, the first signal and the second signal preferably represent different notification or alarm levels depending on how far a pertinent inhalation process parameter is outside the optimal range. A yet further optional functionality of the operation method is the output of a versatile third signal that preferably continuously provides information about the ongoing inhalation process. Unlike the first signal and the second signal that could be seen to be finite signals that only occur under certain conditions, the third signal could be construed to be an informational signal that allows a user/patient to be informed live about one or more pertinent inhalation process parameters as they evolve throughout the ongoing inhalation process. In other words, the third signal continuously informs the user/patient about the specifics and the quality of an ongoing inhalation process.

A wide range of possible signals outputs by the signalling unit is conceivable and part of the present disclosure and invention. For example, such signals may range from simple and different light signals output directly on or through the inhalation device but may also embrace elaborate graphical information or voice information output by an external signalling unit, for example, when implemented in an external electronic device like a smartphone or tablet computer. In the latter case, it is conceivable that there is detailed information related to the ongoing inhalation process and its parameters are output to provide the user/patient or the physician or caregiver with a detailed account of the ongoing inhalation process. Naturally, the information provided can then form the basis for improving the ongoing inhalation processes by instructing the user/patient what to do in order to improve the ongoing inhalation process. Preferably, in the present invention and disclosure, the first signal and the second signal form a functional unit and are interrelated, whereas the third signal may occur unrelated to and independent from the first signal and the second signal. The respective "and/or" connection used above and in original claim 1 do exactly indicate this.

Finally, it is noted that the above and herein below defined operation method relates to the intrinsic functionalities of an inhalation device or components of an inhalation device system. Said operation method is thus preferably independent from an actual inhalation process to be carried out by a user/patient. Thus, the operation method represents a non-therapeutic, non-diagnostic and non-surgical method of operating an inhalation device and thus optionally does not relate to methods for treatment of the human or animal body by surgery or therapy and diagnostic methods practised on the human or animal body.

In the above method, the first signal is a first vibration signal and/or a first light signal and the second signal is a combination of a second vibration signal that is stronger or different to the first vibration signal and a second light signal that is different to the first light signal.

The above is a non-exclusive example of how the above-mentioned signal cascade of the first signal and the second signal may look like in practice. The first signal, i.e., the first alarm signal can be a first vibration signal on its own or in combination with the first light signal. The second signal is then preferably a combination of a second vibration signal that is distinguishable by the user/patient from the first vibration signal and the second light signal that is different from the first light signal. Hence, it is conceivable that the first signal is a "gentle" signal making the user/patient aware that one inhalation process parameter is becoming non-optimal. The second signal is then a more assertive signal that makes the user/patient aware that one or more pertinent inhalation process parameters have become increasingly non-optimal, i.e., worsened with respect to the situation when the first signal was output. It is conceivable and embraced in the present disclosure and invention that the first signal and the second signal and the respective signal cascade can be a combination of any conceivable and suitable signals perceivable by the user/patient. The above example is thus non-limiting. Accordingly, the following signal cascades are also embraced: an optical signal in a first colour and an optical signal in a second colour optionally each in combination with vibration signals, a first vibration signal and a different second vibration signal only, a first combination of a vibration and/or light and/or sound signal and a second combination of a vibration and/or light and/or sound signal.

Preferably the above method further comprises generating and sending a control signal to the signalling unit, wherein in response to said control signal, the signalling unit outputs a fourth signal if and whenever the inhalation device is not ready for use.

This represents an additional functionality of the operating method of the inhalation device, wherein the suitable signal is output and indicates when the inhalation device is not ready for use. This is advantageous in alerting and informing the user about a non-operable state of the inhalation device and potentially errors that need to be mended before use. Preferably, if said fourth signal is not output, then the user/patient may assume an operable state of the inhalation device and commence or continue the inhalation process. As already explained above but also as further explained herein below, said fourth signal can be of any type and any nature as long as it is suitably perceivable by the user/patient. One non-limiting example is the output of a yellow or red light on the housing of the inhalation device itself.

In the operating method according to the present disclosure and invention, preferably the signalling unit outputs the first signal if and whenever the first pre-set threshold value of an inhalation process parameter is exceeded during the inhalation process and outputs the second signal if and whenever the second pre-set threshold value of an inhalation process parameter, that is higher than the first pre-set threshold value, is exceeded during the inhalation process.

This optional scenario relates to cases where pertinent inhalation process parameters should be limited with respect to an upper level that is indicative of a non-optimal inhalation process. In such cases, a first pre-set threshold value exists that if exceeded represents the onset of a non-optimal inhalation process. Accordingly, the first signal is output. There is then a second pre-set threshold value that is higher than the first pre-set threshold value and if said value is exceeded, then this is indicative of a further worsening of the inhalation process with respect to that inhalation process parameters. Consequently, the second signal is output to indicate the growing deterioration of the inhalation process prompting the user/patient to counteract accordingly. Optionally and as explained above, the first signal and the second signal may also be indicative of the degree of deviation from a threshold value. The signal output thus depends on how strongly the threshold value is exceeded. A small exceedance triggers the first signal and a larger exceedance then triggers the second signal.

One non-limiting preferred example of the above scenario is a case where the inhalation process parameter is the breath flow rate and/or the tidal volume during the inhalation process, and optionally wherein the first pre-set threshold value of the breath flow rate is in the range of 30 l/min or more, preferably 40 l/min or more to less than 75 l/min and the second pre-set threshold of the breath flow rate is 75 l/min or more.

In this example of the operation method, the ongoing inhalation process is monitored with respect to the breath flow rate during the inhalation process. If the value of the breath flow rate exceeds the first pre-set threshold value of 30 l/min then the first signal is output as a first information or alarm indicating to the user/patient that the inhalation process is on the onset of becoming non-optimal. Depending on the capabilities of the signalling unit, the user/patient may be directly informed that specifically the breath flow rate is excessive. If the user/patient does not counteract and the inhalation process worsens with respect to the breath flow rate and consequently the second pre-set threshold value of 75 l/min is exceeded, then the second signal is output informing or alerting the user that there is an urgency in improving the ongoing inhalation process. The skilled reader will readily understand that using the breath flow rate or the tidal volume are just two of many possible examples of inhalation process parameters that could be taken into account when issuing the first, second or third signal in the context of the present operation method. The skilled reader will also understand that for certain inhalation process parameters, exceeding upper limits is not crucial, but underrunning lower limits thereof is. In such scenarios the first signal is output if and whenever a first critical threshold value is underrun, meaning that the value of the respective inhalation process parameter is too low. If said value decreases even further and thus underruns the second pre-set threshold value, the second signal is output. A non-limiting example thereof is the tidal volume that can be monitored and considered and a significant pre-set threshold value is 1 liter. If said value is underrun, the user/patient is alerted via a respective signal output and instructed to take deeper breaths through the inhalation device.

It is further optionally embraced that the respective pre-set threshold values are user-specific and are pre-set according to the inhalation specifics of a user/patient. For example, for users/patients who naturally have a high default breath flow rate, the threshold values are set higher than for users/patients that naturally have lower default breath flow rates. This way it can be avoided that the first or second signal is issued inflationary, but instead only when user/patient-specific threshold values are underrun or exceeded.

A further concept of the present invention and disclosure is that the pre-set threshold values are not fixed through the inhalation process but can be changed and adapted during the inhalation process. For example, the first pre-set threshold value and/or the second threshold value at the beginning of the inhalation process may have different values as compared to the middle or the end of the inhalation process. Hence, in particular the first and second signal are then output according to different preconditions at different times during the inhalation process. This may be used for example to adjust the feedback to a reasonable threshold value depending on the current/daily state of lung health, motivation and or wellbeing of the user/patient to allow him to optimize his inhalation in the range within his capabilities and not to frustrate or demotivate the patient/user.

In the above and herein described operation method, preferably the signalling unit comprises or consists of any one of the following: means to generate an optical signal; preferably a light signal, means to produce an acoustic signal; preferably a sound and/or a voice signal, means to produce a tactile signal; preferably a vibration signal and an electronic display device.

It is thus further part of the present disclosure and invention that in the here described methods, processes and systems, a variety of means are conceivable for the signalling unit. Embraced are basically all technical means and hardware that are capable and configured to output signals that are perceivable by humans. These means can also be freely combined in order to output any conceivable signal sequence or signal cascade that can comprise any one of optical signals, acoustic signals and haptic signals.

In one option of the present disclosure and invention, the signalling unit is implemented in the inhalation device itself.

In such a case, the signalling unit is an integral part of the inhalation device itself and thus integrated therein. This in turn implies that the inhalation device itself outputs respective signals and feedback information via the signalling unit comprised therein. For that purpose, the inhalation device itself embraces any suitable hardware means for outputting such signals and information. This has the advantage that the user/patient has a single device with which an inhalation process is performed and that at the same time provides inhalation process feedback and guidance information. For example, the inhalation device may comprise as the signalling unit one or more light sources, for example LEDs that produce light signals representative of the first signal and the second signal or a vibrator that produces a tactile signal.

Based on the above option of having the signalling unit being part of the inhalation device itself, it is preferred that the first signal is a first vibration signal of the inhalation device and/or a first light signal from the inhalation device and that the second signal is a combination of a second vibration signal of the inhalation device that is stronger or different to the first vibration and a second light signal from the inhalation device that is different or identical to the first light signal, optionally wherein the first and the second light signals are output from a top part of the inhalation device, which is made of translucent material through which the light signals are emitted.

This further option underlines that in scenarios where the signalling unit is part of the inhalation device, a versatile number of signals, signal combinations and signal cascades may emanate from the inhalation device itself, which contains the respective means for generating such signals. The inhalation device itself thus conveys the respective information content directly to the user/patient.

A further related option in cases where the signalling unit as part of the inhalation device preferably embraces that the fourth signal is a combination of a third vibration signal of the inhalation device and a sound signal from the inhalation device.

Accordingly, as above, the skilled reader will readily understand that also, for example, the fourth signal can be output and emanated in various suitable forms from the inhalation device itself to convey the respective information content directly to the user/patient. It is especially beneficial to emit such inhalation device status signal also on the device itself, such that a user/patient recognizes it directly in connection with the device itself.

Another option embraced by the present disclosure and invention is that the signalling unit is implemented in a device different from and external to the inhalation device, preferably in an electronic device further preferably with a computer program stored on the electronic device configured to generate a graphical user interface for outputting data of the inhalation process.

In such a case, the respective signals are output and the respective information or alarms and inhalation process/breath guidance are provided on a device that is not the inhalation device itself, but a device separate and external thereto. Preferably envisaged are external electronic devices like mobile phones, smartphones, tablet- and mobile computers and any kind of smart personal electronic devices. All of the devices commonly include electronic display devices, processors and suitable computer programs that enable said devices to output information including the first and second signal, but also additional optional information on the inhalation process. For example, it is inconceivable that on the display devices of the aforementioned electronic devices inhalation process/breath guidance is provided by suitable graphical and/or written information. Also conceivable is a respective output in the form of sound or voice via suitable hardware means related to the electronic- or electronic display device. In principle, inhalation process/breath guidance can be provided by all types of human-perceivable signals that the respective electronic devices can generate.

As part of the present disclosure and invention it is also foreseen that the above options of having an integral and non-integral, i.e., external signalling unit are combinable or combined. For example, it is surely embraced herein that on the one hand the inhalation device itself provides a basic inhalation process/breath guidance, whereas the signalling unit in from of or as part of an external electrical device additionally provides the inhalation process/breath guidance in the same or in a different form and even further details. The latter is due to the fact that the aforementioned electronic devices normally have larger and more versatile signal output capabilities than the ones that could be incorporated within the inhalation device itself. In other words, the signalling unit may optionally be part of the inhalation device and the external device and thus outputs the respective signals on said both devices. Basically, this would imply the provision of two or more signalling devices or one signalling device with two or more sub-units. Finally, it is clear that for the above option where the signalling unit is an external unit that respective technical means are present that allow the exchange of data between the sensing unit, the control unit and the signalling unit irrespectively from where they are located and implemented. All technical means conceivable by the skilled person for said purpose are embraced here.

As an option related to the above case of an external signalling unit, the signalling unit is preferably an electronic display device and the method further comprises: that in response to said control signal and during the inhalation process, the electronic display device outputs, preferably as the third signal, graphically and/or numerically and/or in writing and/or in spoken text at least one of the group of information consisting of: breath pattern, breath flow rate, peak flow rate, regularity of breathing cycle duration, tidal volume, inhalation/exhalation ratio, respiratory minute volume, breathing frequency, breathing durations, breath pauses, live inhalation feedback information and instructions on how to optimize the ongoing inhalation process, wherein the displayed information is to inform a user of the inhalation device about the quality of the inhalation process and to trigger improvements of the inhalation process, and optionally the displaying of information is adjusted or stopped or suspended if and whenever a user of the inhalation device cannot abide to improve the inhalation process. Further optionally, the displaying of information is sorted according to the most important to the least important inhalation process parameters to be changed or improved.

As it can be appreciated from the above outlined optional aspects of the present operation method, in particular when an external signalling unit is outputting the respective signals, a large number of additional functionalities of the operation method are conceivable. For example, the values of various different inhalation process parameters as listed above can be continuously displayed so as to give the user/patient or an accompanying physician or caregiver live insights into the ongoing inhalation process. Related to said live output, the operation method may further comprise the output of additional information for the user/patient how to improve the ongoing inhalation process with respect to one or more relevant inhalation process parameters. Hence, the user/patient is basically given specific proposals and recommendations on how to improve the ongoing inhalation process. The user/patient is thus further given the chance to improve the inhalation process as it occurs and as it is ongoing. However, as the skilled reader may appreciate, a user/patient may not always be able or willing to follow such instructions due to his individual state of disease or due to his daily wellbeing or due to his emotional and/or mental state or due to his cognitive capabilities and to improve an ongoing inhalation process. In such cases a continuous output of notifications, information or alarms or relevant live data and improvement instructions may become frustrating for the user/patient. Consequently, it is also embraced by the present disclosure and invention that respective signal outputs are muted, suspended or completely stopped if the user/patient is not in a position to improve the ongoing inhalation process with respect to one or more pertinent inhalation process parameters. The skilled reader will appreciate that this functionality is based on a continuous monitoring of the inhalation process and in particular relating to the fact that threshold values for certain inhalation parameters are underrun or exceeded with potential changes in inhalation behaviour of a user/patient. If during an ongoing non-optimal inhalation process the monitoring yields that for a certain amount of time the user/patient is not able to improve the inhalation process, then the respective signal outputs are muted, stopped or suspended. In the above case, the information output by the optional electronic device or electronic display device are part of or constitute the third signal. Alternatively, in such a situation, the pre-set threshold values can be adapted to adjust to the user's/patient's daily status. In an alternative example, the thresholds are adapted stepwise while monitoring and guiding the user/patient to consider a stepwise guiding towards an optimal inhalation manoeuvre.

Said further examples of the present disclosure and invention highlight again that in particular in the case where the signalling unit is an external unit and the signal output is performed on said external unit which is preferably an electronic device and more preferably an electronic display device, the degrees of freedom of the signals output are thus high. Hence, the various signals outlined herein can be elaborate signals and clearly go beyond simple light or sound signals and can basically embrace any suitable information output within the technical capabilities of an electronic display device or more generally an electronic device including an electronic display device but also including respective other signal devices, for example standard light devices.

The above listed inhalation process parameters represent parameters that the sensing unit is configured to measure and monitor and are in line with the skilled person's common technical understanding of the respective terminologies used. Based on one or more of said parameters, the above-described inhalation process and breath guiding is generated and given. It is embraced by the present disclosure and invention that one or more or all of the above-indicated parameters can be considered in the process of evaluating the quality of an ongoing inhalation process and outputting the respective signals and inhalation process information and guidance. Also, for each of said inhalation process parameters individual pre-set threshold values may be selected that then in turn determine the output scenarios in particular for the first signal and the second signal.

Especially for the output on or via an external electronic device where there are more possibilities of displaying output information, a selection of which parameters are to be guided first can be implemented to first guide the user/patient on the most important parameter and when this parameter is below/above the pre-set threshold value, the next parameter can be guided to stepwise lead the patient/user to an optimal breathing manoeuvre/process during his inhalation.

It can also be considered to implement positive feedback on such an electronic device, if the user/patient was successful in performing his/her inhalation within the pre-set thresholds and also to give positive feedback when he/she managed to change his inhalation process and do it within the pre-set threshold values after he was informed about being above the pre-set threshold values.

In line with the above outlined additional functionalities, it is preferred that the first signal and the second signal inform the user/patient of the inhalation device about a non-optimal inhalation process and trigger improvements of the inhalation process, and/or the first signal and the second signal are stopped or adjusted, muted or suspended if a user of the inhalation device cannot abide to improve the inhalation process.

This option basically epitomizes the technical concepts and functionalities already described herein above and underlines that in particular the first signal and the second signal could be seen to be signals that inform or alert the user/patient about a non-optimal inhalation process. Said signals could then also prompt the user/patient to initiate means for improving the ongoing inhalation process. Should the user/patient not be in the position for that or unwilling to do so, the respective signals can be stopped, muted or suspended in order not to frustrate, annoy or unsettle the user/patient.

A further important aspect of the present disclosure and invention is an inhalation device itself comprising: a sensing unit configured to monitor an inhalation process and to measure and collect data of and during the inhalation process, a signalling unit configured to output a signal, preferably an optical, acoustic and/or a tactile signal, a control unit configured to receive and process the data collected by the sensing unit and to control the signalling unit based on said data, wherein the control unit is further configured to control the signalling unit such that the signalling unit outputs a first signal if and whenever a first pre-set threshold value of an inhalation process parameter is underrun or exceeded during the inhalation process and outputs a second signal if and whenever a second pre-set threshold value of an inhalation process parameter, that is different from the first pre-set threshold value, is underrun or exceeded during the inhalation process. The "first signal" and "second signal" are preferably the "first signal" and the "second signal" of the above operation method.

Optionally, the above defined inhalation device is the inhalation device used in the methods and procedures according to the present disclosure and invention, preferably the above operation method. In particular so for the scenario where a signalling unit is incorporated in the inhalation device itself. As it can be appreciated from the above defined inhalation device, preferably all of the respective components, namely the sensing unit, the control unit and the signalling unit are part of the inhalation device itself and comprised therein. Consequently, the inhalation device itself is configured and capable to generate and output the first and the second signal and to provide at least basic inhalation process/breath guidance. Furthermore, if the methods and procedures according to the present disclosure and invention are carried out using the above defined inhalation device, the respective signal output is performed by the inhalation device itself. Basically and optimally, the inhalation device defined herein provides the hardware basis or hardware equivalent of the operation method previously outlined. Consequently, similar and related hardware features are thus found with respect to the present inhalation device and comprise the following ones outlined below, which are preferably mutually combinable. Thereby, the inhalation device embodies the same functionalities and technical advantages that have been explained and outlined above for the operation method.

In one preferred embodiment of the inhalation device the first signal is a first vibration signal of the inhalation device and/or a first light signal and the second signal is a combination of a second vibration signal of the inhalation device that is stronger or different to the first vibration and a second light signal from the inhalation device, optionally wherein the first and the second light signals come from a top part of the inhalation device, which is made of translucent material through which the light signals are emitted. This option is analogous to the above-described option for the operating method and the explanations provided there apply mutatis mutandis here.

In a yet further preferred embodiment of the inhalation device, the control unit is further configured to control the signalling unit such that the signalling unit outputs the first signal if and whenever the first pre-set threshold value of an inhalation process parameter is exceeded during the inhalation and outputs the second signal if and whenever the second pre-set threshold value of an inhalation process parameter, that is higher than the first pre-set threshold value, is exceeded during the inhalation. This option is also analogous to the above-described option for the operating method and the explanations provided there apply mutatis mutandis here.

Optionally, in the inhalation device the inhalation process parameter is the breath flow rate and optionally the tidal volume, and optionally wherein the first pre-set threshold of the breath flow rate is in the range of 30 l/min or more preferably 40 l/min or more to less than 75 l/min and the second pre-set threshold of the breath flow rate is 75 l/min or more. This option is analogous to the above-described option for the operating method and the explanations provided there apply mutatis mutandis here.

Yet further optionally, in the inhalation device the control unit is further configured to control the signalling unit to output a third signal if and whenever the inhalation device is not ready for use, optionally wherein the third signal is a combination of a third vibration signal of the inhalation device and a sound signal from the inhalation device. This option is analogous to the above-described option for the operating method and the explanations provided there apply mutatis mutandis here. The "third signal" here equals the "fourth signal" of the above operation method.

Finally, it is further optional that in the inhalation device the signalling unit comprises or consists of any one of the following accommodated within the inhalation device: means to generate an optical signal; preferably a light signal, means to produce an acoustic signal; preferably a sound signal and/or a voice signal, means to produce a tactile signal; preferably a vibration signal and an electronic display device. This option is also analogous to the above-described option for the operating method and the explanations provided there apply mutatis mutandis here.

A yet further important aspect of the present disclosure and invention is an inhalation device system, comprising: an inhalation device, an electronic device that is different from and separate to the inhalation device, preferably a mobile phone, smartphone, smart watch, smart personal device or tablet computer, wherein both the inhalation device and the electronic device comprise communication means configured to establish a wired or wireless communication connection between the inhalation device and the electronic device to exchange data on an inhalation process performed with the inhalation device ideally in near to real time.

Optionally, the above defined inhalation device system comprises a medical inhalation device and preferably the inhalation device that has been outlined herein above. Yet further optionally, the above-defined inhalation device system is a device-related representation of the above described option of having the signalling unit external to the inhalation device itself. Also, all of the above outlined and explained interrelations, functionalities and technical advantages and benefits apply mutatis mutandis to the inhalation device system and will not be repeated here for the sake of conciseness. The present inhalation device system basically represents a pairing of an inhalation device with which the respiratory treatment, i.e., the inhalation process is performed and an external electronic device that are mutually configured to communicate with each other, such that inhalation process/breath guiding can be provided also on said electronic device. The technical means to allow such mutual communication are the ones that are known to the skilled person and embrace wired and wireless communication means, like data-transfer cables, Bluetooth, NFC or Wi-Fi hardware or the like. As part of the present disclosure and invention and preferably in the context of the present inhalation device system, it is also conceivable that the first to fourth signal and/or inhalation process/breath guidance is provided by means of e-mail or push-notification handled by the external electronic device.

The present disclosure and invention is not limited to the above defined methods and procedures of operating an inhalation device. The present disclosure and optionally the present invention also include an inhalation process in relation thereto. Said inhalation process comprises the following steps: performing an inhalation process preferably to prevent or treat a respiratory disease or malfunction but also for wellbeing and prophylaxis, using an inhalation device, monitoring the ongoing inhalation process with respect to pertinent inhalation process parameters and features and collecting respective data, evaluating the inhalation process based on the monitoring results and the data obtained and collected during the inhalation process and outputting inhalation process information that inform the user/patient and or a physician or caregiver about the current/live quality of the inhalation process via suitable signals constituting the inhalation process information and optionally issuing notifications or alarms if and whenever relevant inhalation process parameters are indicative of a non-optimal inhalation process. Optionally, the notifications, information or alarms can be provided in a cascading/manner where the assertiveness of the notifications, information and alarms is increased as the inhalation process further deviates from an optimal inhalation process, i.e., worsens. Optionally, the inhalation process comprises the output of inhalation process/breath guidance information, which is bespoke information that is issued in response to the monitoring results and the alarms or notifications, information or alarms output to provide the user/patient with recommendations how to improve the ongoing inhalation process. Hence, the user/patient is basically accompanied in his or her inhalation process and given live information on the one hand with respect to basic information of the inhalation process but also in particular with information in case the inhalation process is becoming the non-optimal. In the latter case, not only will the user/patient be informed or alerted, but he or she is also incited to carry out certain actions to improve the ongoing inhalation process. Should the user/patient not be able or willing to improve the inhalation process accordingly, then optionally the respective alarms, notification or information can be stopped or suspended or muted or the parameter threshold values adjusted. This innovative inhalation process procedure basically guides the user/patient using a medical inhalation device through the inhalation process to ensure that inhalation process is performed as good as possible and to trigger means to improve an inhalation process that becomes non-optimal. This way the inhalation process quality can be greatly improved, thus the therapy success is increased and the user/patient experience improved in that the user/patient is not left alone doing inhalation process but guided throughout which improves the user/patient's confidence and satisfaction when performing the inhalation process and using the medical inhalation device.

A yet further aspect of the present disclosure and invention is a computer program that is configured to facilitate the herein described methods and procedures. Specifically embraced is a computer program or app, respectively one that is to be executed on respective means, i.e., computer processors of the inhalation device itself, but preferably of the external electronic device and configured to output the described signals, information and inhalation process/breath guidance in any suitable form and in different degrees of detail. More specifically, the computer program is configured to output the inhalation process/breath guiding information on an electronic display device in graphical form, for instance via generating a suitable graphical user interface, also in line with the examples given above and herein below. Further embraced is a computer-readable storage medium on which the above computer program is stored and yet further a carrier containing the computer program, wherein the carrier is one of an electrical signal, optical signal, radio signal or computer-readable storage medium.

### EXAMPLES

In a preferred example of the present disclosure and invention, the inhalation device is a compact handheld medical device in which the signalling unit is integrated in the form of multi-colour light sources, preferably one or more LEDs and a vibrator. The ongoing inhalation process of the user/patient is monitored by the sensing unit and its related hardware components and the data obtained processed by the control unit. If, for example, the present breath flow rate momentarily exceeds the first pre-set threshold value of 30 l/min or preferably 40 l/min, the control unit triggers the signalling unit to output a first short vibration signal. This signal will be felt by the user/patient, making him or her aware that the ongoing inhalation process is non-optimal. If the user/patient continues the inhalation process and further increase the breath flow rate, for example, beyond the second pre-set threshold value of 75 l/min, the control unit triggers the signalling unit to output the second signal in the form of a stronger or longer vibration signal in combination with an alerting light signal from a transparent top region of the inhalation device, for example, a yellow or red light signal. The user/patient is thus alerted that the quality of the ongoing inhalation process has worsened. Optionally, the first vibration signal may already be accompanied by a yellow or red light signal to avoid that the user/patient misunderstands the first vibration signal to be of positive nature. Also, the first and second signal may simply be indicative of how the user/patient exceeds an optimal breath flow rate. If and whenever the user/patient exceeds the first threshold of 30 or 40 l/min, the first signal is output. If and whenever the degree of deviation is larger, for example, a breath flow rate of 75 l/min or more, the second signal is output. Further optionally, the threshold values are adaptable, for example, to the default breathing and inhalation of individual users/patients or to the default breathing and inhalation of the current inhalation session based on the individual daily characteristics and wellbeing of the patient/user and the course of the inhalation process.

By means of the respective alarms given, the user/patient obtains a chance to improve the inhalation process by adapting his or her inhalation actions. The exemplified first signal and second signal herein basically represent a signal cascade or an alarm cascade to make the user/patient aware of deficits in the ongoing inhalation process. The fourth signal being indicative of the inhalation device involved being inoperable can in the present example be output by a red light signal emanating from the housing of the inhalation device. As another parameter, the tidal volume can be considered and a significant pre-set threshold value is 1. If said value is underrun, the user/patient is alerted and instructed to take deeper breath through the inhalation device.

A yet further non-limiting example of the present disclosure and invention is either separate or combinable with the above example and relates to the option of having the signalling unit external and integrated into an external electronic device consisting of or comprising an electronic display device. In this scenario, once again, an ongoing inhalation process is monitored by the sensing unit, the data obtained and processed by the control unit and the signalling unit is controlled by the control unit to output respective signals. In the present example, an external signalling unit can have an output similar as described above, where the external electronic device outputs a first signal representative of a first alarm and a second signal representative of a second alarm. It is within the nature of the electronic device or the electronic display device that the respective signals can come in many different forms and do not necessarily need to be limited to a combination of vibration and light signals. Instead, it is conceivable that the respective alarms are output by suitable graphical, written and/or voice information. The respective alarms could be output also in addition to any type of alarm output directly on the inhalation device. In the present example, the external signalling unit can also output the third signal in any conceivable form to illustrate relevant parameters of the ongoing inhalation process. As a specific example, the external signalling unit can display a schematic lung illustration that in turn via animations can illustrate the depth and quality of the inhalation live and continuously as the inhalation process progresses. In the present example, the external signalling unit is configured to and outputs inhalation process/breath guiding information to the user/patient that contain recommendations and instructions on how to improve and ongoing inhalation process also with respect to any possible alarm previously output.

## Claims

1. A method of operating an inhalation device that includes:
a sensing unit configured to monitor an inhalation process, to measure inhalation process parameters and to collect data of and during the inhalation process, and
a control unit configured to receive and process the data collected by the sensing unit and to generate a control signal for a signalling device, wherein the method comprises: monitoring an inhalation process comprising measuring inhalation process parameters and collecting data of and during the inhalation process,
generating and sending a control signal based on the collected data to a signalling unit configured to output a signal, preferably an optical, acoustic and/or a tactile signal, wherein
in response to said control signal, the signalling unit outputs:
a first signal if and whenever a first pre-set threshold value of an inhalation process parameter is underrun or exceeded during the inhalation process and
a second signal if and whenever a second pre-set threshold value of an inhalation process parameter, that is different from the first pre-set threshold value, is underrun or exceeded during the inhalation process, and/or
a third signal that is indicative of one or more inhalation process parameters and continuously output during part or all of the inhalation process.

2. The method according to claim 1, wherein
the first signal is a first vibration signal and/or a first light signal and
the second signal is a combination of a second vibration signal that is stronger or different to the first vibration signal and a second light signal that is different to the first light signal.

3. The method according to claim 1 or 2, further comprising
generating and sending a control signal to the signalling unit, wherein
in response to said control signal, the signalling unit outputs a fourth signal if and whenever the inhalation device is not ready for use.

4. The method according to any one of the preceding claims, wherein
the signalling unit outputs the first signal if and whenever the first pre-set threshold value of an inhalation process parameter is exceeded during the inhalation process and
outputs the second signal if and whenever the second pre-set threshold value of an inhalation process parameter, that is higher than the first pre-set threshold value, is exceeded during the inhalation process.

5. The method according to claim 4, wherein
the inhalation process parameter is the breath flow rate and/or the tidal volume, and optionally wherein
the first pre-set threshold of the breath flow rate is in the range of 30 l/min or more, preferably 40 l/min or more to less than 75 l/min and the second pre-set threshold of the breath flow rate is 75 l/min or more.

6. The method according to any one of the preceding claims, wherein
the signalling unit comprises or consists of any one of the following: means to generate an optical signal; preferably a light signal, means to produce an acoustic signal; preferably a sound and/or a voice signal, means to produce a tactile signal; preferably a vibration signal and an electronic display device.

7. The method according to any one of claims 1 to 6, wherein
the signalling unit is implemented in the inhalation device itself.

8. The method according to claim 7, wherein
the first signal is a first vibration signal of the inhalation device and /or a first light signal from the inhalation device and
the second signal is a combination of a second vibration signal of the inhalation device that is stronger or different to the first vibration and a second light signal from the inhalation device that is different to the first light signal, optionally wherein
the first and the second light signals are output from a top part of the inhalation device, which is made of translucent material through which the light signals are emitted.

9. The method according to claim 7 or 8, wherein
the fourth signal is a combination of a third vibration signal of the inhalation device and a sound signal from the inhalation device.

10. The method according to any one of claims 1 to 6, wherein
the signalling unit is implemented in a device different from and external to the inhalation device, preferably
in an electronic device further preferably with a computer program stored on the electronic device configured to generate a graphical user interface for outputting data of the inhalation process.

11. The method according to claim 10, wherein
the signalling unit is an electronic display device and the method further comprises:
in response to said control signal and during the inhalation process, the electronic display device outputs, preferably as the third signal, graphically and/or numerically and/or in writing and/or in written and spoken text at least one of the group of information consisting of: breath pattern, breath flow rate, peak flow rate, regularity of breathing cycles duration, tidal volume, inhalation/exhalation ratio, respiratory minute volume, breathing frequency, breathing durations, breath pauses live inhalation feedback information and instructions on how to optimize the ongoing inhalation process, wherein
the displayed information is to inform a user of the inhalation device about the quality of the inhalation process and to trigger improvements of the inhalation process, and optionally
the displaying of information is adjusted or stopped or suspended if and whenever a user of the inhalation device cannot abide to improve the inhalation process, and further optionally
the displaying of information is sorted according to the most important to the least important inhalation process parameters to be changed or improved.

12. The method according to any one of the preceding claims, wherein
the first signal and the second signal inform the user of the inhalation device about a non-optimal inhalation process and to trigger improvements of the inhalation process, and/or
the first signal and the second signal are stopped or adjusted if a user of the inhalation device cannot abide to improve the inhalation process.

13. An inhalation device, optionally the one to carry out the method according to any one of claims 1 to 9, comprising:
a sensing unit configured to monitor an inhalation process and to measure and collect data of and during the inhalation process,
a signalling unit configured to output a signal, preferably an optical, acoustic and/or a tactile signal,
a control unit configured to receive and process the data collected by the sensing unit and to control the signalling unit based on said data, wherein
the control unit is further configured to control the signalling unit such that the signalling unit outputs a first signal if and whenever a first pre-set threshold value of an inhalation process parameter is underrun or exceeded during the inhalation process and
outputs a second signal if and whenever a second pre-set threshold value of an inhalation process parameter, that is different from the first pre-set threshold value, is underrun or exceeded during the inhalation process.

14. The inhalation device according to claim 13, wherein
the first signal is a first vibration signal of the inhalation device and or a first light signal and the second signal is a combination of a second vibration signal of the inhalation device that is stronger or different to the first vibration and a second light signal from the inhalation device, optionally wherein
the first and the second light signals comes from a top part of the inhalation device, which is made of translucent material through which the light signals are emitted.

15. The inhalation device according to claim 13 or 14,
wherein
the control unit is further configured to control the signalling unit such that the signalling unit outputs the first signal if and whenever the first pre-set threshold value of an inhalation process parameter is exceeded during the inhalation and
outputs the second signal if and whenever the second pre-set threshold value of an inhalation process parameter, that is higher than the first pre-set threshold value, is exceeded during the inhalation.

16. The inhalation device according to claim 15, wherein
the inhalation process parameter is the breath flow rate and optionally the tidal volume, and optionally wherein
the first pre-set threshold of the breath flow rate is in the range of 30 l/min or more preferably 40 l/min or more to less than 75 l/min and the second pre-set threshold of the breath flow rate is 75 l/min or more.

17. The inhalation device according to any one of the preceding claims 13 to 16, wherein
the control unit is further configured to control the signalling unit to output a third signal if and whenever the inhalation device is not ready for use, optionally wherein
the third signal is a combination of a third vibration signal of the inhalation device and a sound signal from the inhalation device.

18. The inhalation device according to any one of the preceding claims 13 to 17, wherein
the signalling unit comprises or consists of any one of the following accommodated within the inhalation device: means to generate an optical signal; preferably a light signal, means to produce an acoustic signal; preferably a sound signal and/or a voice signal, means to produce a tactile signal; preferably a vibration signal and an electronic display device.

19. An inhalation device system, comprising:
an inhalation device, preferably the inhalation device as used in any one of claims 1 to 12 or the inhalation device according to any one of claims 13 to 18 and
an electronic device that is different from and separate to the inhalation device, preferably a mobile phone, smartphone, smart watch, smart personal device or tablet computer, wherein
both the inhalation device and the electronic device comprise communication means, configured to establish a wired or wireless communication connection between the inhalation device and the electronic device to exchange data on an inhalation process performed with the inhalation device.
